# EUROPÄISCHE PATENTSCHRIFT

(11) **EP 0 355 562 B1**
(45) Veröffentlichungstag und Bekanntmachung des Hinweises auf die Patenterteilung: **05.01.1994**
(21) Anmeldenummer: 89114607.8
(22) Anmeldetag: 08.08.1989
(51) Int. Cl.: C08F 20/36, C09J 133/14, A61K 6/00, A61L 25/00

(54) **Adhäsivkomponente zur Behandlung kollagenhaltiger Materialien**
Adhesive for treating collageneous material
Adhésif pour le traitement de matériaux contenant du collagène

(30) Priorität: 19.08.1988 DE 3828169
(43) Veröffentlichungstag der Anmeldung: 28.02.1990
(73) Patentinhaber: BAYER AG, 51368 Leverkusen (DE)
(72) Erfinder: Müller, Michael, Dr., D-5060 Bergisch-Gladbach 2 (DE); Podszun, Wolfgang, Dr., D-5000 Köln 80 (DE); Alker, Bernd, D-5060 Bergisch Gladbach (DE)

(56) Entgegenhaltungen:
- DE-A- 2 507 189
- FR-A- 1 410 558
- US-A- 4 404 327

## Beschreibung

Die Erfindung betrifft Zubereitungen zur Verwendung als Adhäsivkomponente zur Behandlung kollagenhaltiger Materialien, ein Verfahren zu ihrer Herstellung und ihre Verwendung.

Kollagenhaltige Materialien sind Gerüsteiweißkörper und Hauptbestandteile der menschlichen und tierischen interzellularen Stützsubstanzen wie Knorpel- und Knochengewebe, Haut und Zahnbein (Dentin). Im Rahmen der vorliegenden Erfindung werden die Adhäsivkomponenten bevorzugt zur Behandlung von Dentin im Zusammenhang mit Zahnreparaturen verwendet.

Besonders im Dentalbereich werden härtende, polymere Materialien als Füllmaterialien bei Zahnreparaturen verwendet. Als härtende, polymere Materialien werden im allgemeinen Füllungen auf Acrylatbasis bevorzugt. Diese polymeren Füllungen haben jedoch den Nachteil, daß sie schlecht am Dentin haften bleiben. Um dieses Problem zu lösen, hat man bisher teilweise Unterschneidungen am Zahnbeim vorgenommen; dazu war es erforderlich, über den angegriffenen Bereich hinaus, beachtliche Mengen an frischem Dentin zu entfernen.

Nach einer anderen Methode ätzt man das Dentin und die Schmelzoberfläche mit Säuren, wie z.B. Phosphorsäure, an, und nimmt dann die Füllung vor. Abgesehen davon, daß die Säure eine Reizwirkung im Mundbereich ausübt, dringt sie auch leicht durch die Dentinkanäle in den Zahn und schädigt den Nerv (Pulpa).

In J. Dent. Res. 57, 500-505 (1978), werden aldehydgruppenhaltige Methacrylate der isomeren Hydroxybenzaldehyde beschrieben, die als Grundierungsmittel für Füllungen im Dentalbereich verwendet werden können. Die Bindung zwischen Dentin und Füllmasse bleibt jedoch auch nach einer solchen Grundierung unbefriedigend.

In Scand. J. Dent. Res. 92, 980-983 (1984) und J. Dent. Res. 63, 1087-1089 (1984) werden Grundierungsmittel aus wäßrigem Formaldehyd oder Glutaraldehyd und β-Hydroxy-ethylmethacrylat (HEMA) beschrieben.

Außerdem sind in der EP-A-0 141 324 Zusammensetzungen aus einem Aldehyd und einem olefinisch ungesättigten Monomer mit aktivem Wasserstoff beschrieben, die eine gute Anbindung an Dentin aufweisen.

Es wurden Adhäsiv-Zubereitungen, enthaltend Formamidgruppen enthaltende (Meth)-acrylsäureester der Formel 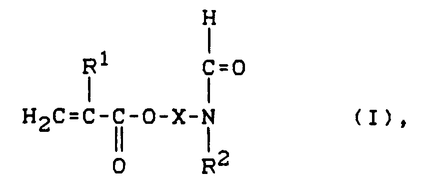
in der
- R¹: Wasserstoff oder Methyl bedeutet,
- R²: Wasserstoff, gegebenenfalls durch Hydroxy, Carboxy, Halogen oder Amino der Formel 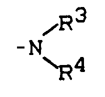 in der
- R³ und R⁴: gleich oder verschieden sind und Wasserstoff oder Niederalkyl bedeuten,
substituiertes Alkyl (C₁ bis C₁₂), Aryl (C₆ bis C₁₂) oder Aralkyl (C₇ bis C₁₄) bedeutet, und
- X: ein zweiwertiger, gegebenenfalls durch Hydroxy, Carboxy, Halogen oder Amino der Formel 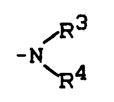 in der
- R³ und R⁴: die obengenannte Bedeutung haben, substituierter aliphatischer (C₁ bis C₂₄) und/oder cycloaliphatischer (C₅ bis C₈) und/oder aromatischer (C₆ bis C₁₂) Rest, der gegebenenfalls eine oder mehrere Sauerstoff-, Schwefel- und/oder -NR³-Brücken enthalten kann,
wobei
- R³: die obengenannte Bedeutung hat,
bedeutet,
und gegebenenfalls Initiatoren zur Anwendung in einem Verfahren zur therapeutischen Behandlung kollagenhaltiger, menschlicher und tierischer interzellularer Stützsubstanzen. gefunden.

Bevorzugt werden erfindungsgemäß (Meth)-acrylsäureester der Formel (I) eingesetzt,
in welcher
- R¹: Wasserstoff oder Methyl bedeutet,
- R²: Wasserstoff, gegebenenfalls durch Hydroxy, Carboxy, Fluor, Chlor oder Amino substituiertes Alkyl (C₁ bis C₆), Phenyl, Tolyl, Benzyl oder Methyl substituiertes Benzyl bedeutet, und
- X: einen zweiwertigen, gegebenenfalls durch Hydroxy, Carboxy, Fluor, Chlor oder Amino substituierten, aliphatischen (C₁ bis C₂₀) und/oder cycloaliphatischen (C₅ und C₆) und/oder aromatischen (C₆ bis C₁₂)-Rest, der gegebenenfalls ein bis zehn Sauerstoff- oder -NR³-Brücken enthalten kann,
wobei R³ die obengenannte Bedeutung hat,
bedeutet.

Besonders bevorzugt werden erfindungsgemäß (Meth)-acrylsäureester der Formel (I) eingesetzt,
worin
- R¹: Wasserstoff oder Methyl bedeutet,
- R²: Wasserstoff, gegebenenfalls durch Hydroxy, Carboxy, Fluor, Chlor oder Amino substituiertes Alkyl (C₁ bis C₆), Phenyl oder Tolyl bedeutet, und
- X: einen zweiwertigen, gegebenenfalls durch Hydroxy, Carboxy, Fluor, Chlor oder Amino substituierten, aliphatischen (C₁ bis C₈)- und/oder cycloaliphatischen (C₅ und C₆)- und/oder Phenylen-Rest, der gegebenenfalls ein bis vier Sauerstoffbrücken enthalten kann, bedeutet.

Die neuen Zubereitungen bewirken eine starke Verbundhaftung von Materialien, die am Kollagen befestigt werden sollen, z.B. eine Verbundhaftung von Zahnfüllungsmaterial in einer Kavität am Zahn.

Formamidgruppen enthaltende (Meth)-acrylsäureester sind aus der DE-A-2 507 189 bekannt. In der DE-A-2 507 189 wird auch beschrieben, diese Acrylsäureester als Überzüge oder Klebemittel für Papier und Textilien zu verwenden. Die Verwendung der erfindungsgemäßen Formamidgruppen enthaltenden (Meth)-acrylsäureester als Adhäsivkomponente für kollagenhaltige Materialien war überraschend, weil sie keine reaktiven Gruppen enthalten, die unter milden Bedingungen geeignete chemische Bindungen zu kollagenhaltigen Materialien aufbauen könnten.

(Meth)-acrylsäureester im Rahmen der vorliegenden Erfindung sind die Ester der Acrylsäure und der Meth-acrylsäure.

Die Substituenten der erfindungsgemäßen (Meth)-acrylsäureester im Rahmen der allgemeinen Formel (I) haben im allgemeinen die folgende Bedeutung:
Halogen bedeutet im allgemeinen Fluor, Chlor, Brom und Iod, bevorzugt Fluor und Chlor.

Niederalkyl (R³ und R⁴) im Rahmen der Aminogruppen bedeutet im allgemeinen einen geradkettigen oder verzweigten Kohlenwasserstoffrest mit 1 bis etwa 6 Kohlenstoffatomen. Bevorzugt ist Methyl und Ethyl.

Als Aminogruppen seien beispielsweise Amino, Dimethylamino, Diethylamino, Methyl-ethyl-amino,
genannt.

Alkyl (R²) bedeutet im allgemeinen einen geradkettigen oder verzweigten Kohlenwasserstoffrest mit 1 bis 12, bevorzugt 1 bis 6, Kohlenstoffatomen. Beispielsweise seien die folgenden Alkylreste genannt: Methyl, Ethyl, Propyl, Isopropyl, Butyl, Isobutyl, Pentyl, Isopentyl, Hexyl und Isohexyl. Besonders bevorzugt werden Methyl und Ethyl.

Aryl bedeutet im allgemeinen einen aromatischen Kohlenwasserstoffrest, der gegebenenfalls auch substituiert sein kann (z.B. durch Niederalkyl), mit 6 bis 12 Kohlenstoffatomen. Beispielsweise seien die folgenden Arylreste genannt: Phenyl, Diphenyl, Naphthyl und Tolyl. Bevorzugt werden Phenyl und Tolyl.

Aralkyl bedeutet im allgemeinen einen über eine Alkylenkette gebundenen Arylrest mit 7 bis 14 Kohlenstoffatomen, der gegebenenfalls auch substituiert sein kann (beispielsweise durch Niederalkyl). Bevorzugt werden Aralkylreste mit 1 bis 6 Kohlenstoffatomen im aliphatischen Teil und 6 bis 12 Kohlenstoffatomen im aromatischen Teil. Beispielsweise seien die folgenden Aralkylreste genannt: Benzyl, Naphthyl/methyl, Phenethyl, Phenylpropyl und durch Methyl substituiertes Benzyl. Bevorzugt werden Benzyl und durch Methyl substituiertes Benzyl.

Ein zweiwertiger aliphatischer Rest (X) bedeutet im allgemeinen einen zweiwertigen, geradkettigen oder verzweigten Kohlenwasserstoffrest mit 1 bis 24, bevorzugt 1 bis 20, insbesondere bevorzugt 1 bis 10, Kohlenstoffatomen. Beispielsweise seien die folgenden zweiwertigen aliphatischen Reste genannt: Dodecandiyl, Undecandiyl, Decandiyl, Nonandiyl, Octandiyl, Heptandiyl, Hexandiyl, 2,3-Dimethylbutandiyl, Pentandiyl, Neopentandiyl, Butandiyl, Dimethylethandiyl, Propandiyl, Ethandiyl.

Bevorzugt werden als zweiwertige aliphatische Reste Ethandiyl und Propandiyl.

Ein zweiwertiger cycloaliphatischer Rest (X) bedeutet im allgemeinen einen cyclischen Kohlenwasserstoffrest mit 5 bis 8, bevorzugt 5 oder 6, Kohlenstoffatomen. Bei spielsweise seien die folgenden zweiwertigen cycloaliphatischen Reste genannt: Cyclopentyl, Cyclohexyl, Cycloheptyl und Cyclooctyl. Bevorzugt werden Cyclopentyl und Cyclohexyl.

Ein zweiwertiger aromatischer Rest (X) bedeutet im allgemeinen einen zweiwertigen aromatischen Kohlenwasserstoffrest mit 6 bis 12 Kohlenstoffatomen. Beispielsweise seien die folgenden zweiwertigen aromatischen Reste genannt: Phenylen, Biphenyldiyl und Naphthylendiyl. Bevorzugt wird Phenylen.

Die zweiwertigen aliphatischen, cycloaliphatischen und aromatischen Reste können durch Hydroxy, Carboxy, Halogen oder Aminogruppen substituiert sein. Bevorzugt werden als Substituenten Hydroxy, Carboxy, Fluor, Chlor und Amino. X kann im allgemeinen durch 1 bis 10 Reste substituiert sein.

Es ist aber auch möglich, daß X aus aliphatischen und cycloaliphatischen, aliphatischen und aromatischen, cycloaliphatischen und aromatischen, oder aliphatischen, cycloaliphatischen und aromatischen Resten besteht. Hierbei liegt die gesamte Zahl der Kohlenstoffatome im Bereich von 6 bis 30, bevorzugt von 6 bis 15. Bevorzugt werden zweiwertige Reste, bei denen Phenylenreste über Methylenbrücken gebunden sind.

Es ist aber auch möglich, daß in X die aliphatischen, cycloaliphatischen und aromatischen Reste durch Sauerstoff, Schwefel und/oder -NR³-Brücken (wobei R³ die obengenannte Bedeutung hat) verbunden sind. Hierbei ist es möglich, daß die aliphatischen, cycloaliphatischen und aromatischen Reste jeweils nur durch Sauerstoff oder Schwefel oder -NR³- verbunden sind. Es ist aber auch möglich, daß verschiedene Brückenglieder die aliphatischen, cycloaliphatischen und aromatischen Reste miteinander verbinden. Hierbei liegt die Gesamtzahl der Kohlenstoffatome im Bereich von 4 bis 45, bevorzugt von 4 bis 12, und die Zahl der Brückenglieder im Bereich von 1 bis 20, bevorzugt von 1 bis 6.

Bevorzugt werden zweiwertige Reste, bei denen Ethylenreste über Sauerstoffbrücken gebunden sind.

Beispielsweise seien die folgenden Formamidgruppen enthaltenden (Meth)-acrylsäureester genannt: 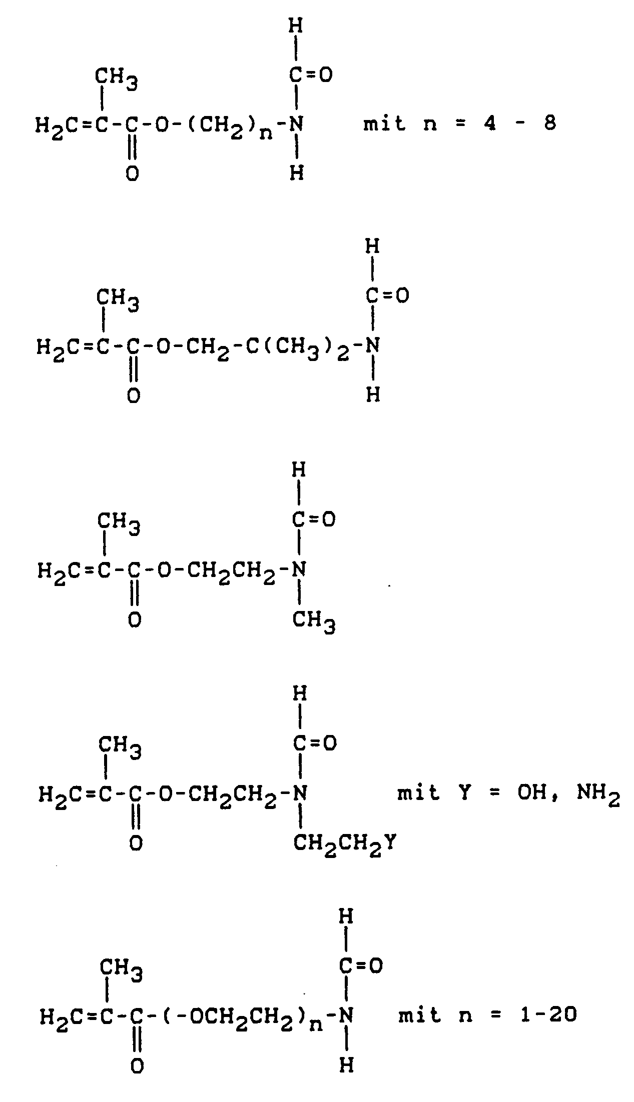
Besonders bevorzugt sind Methacrylsäure-3-formamidopropylester und Methacrylsäure-2-formamidoethylester der Formel

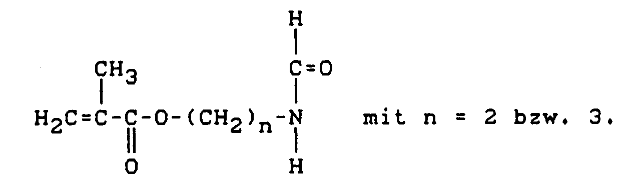

Die Herstellung der erfindungsgemäßen Formamidgruppen enthaltenden (Meth)-acrylsäureester ist an sich bekannt (DE-A-1 770 964 und DE-A-2 507 189). Beispielsweise können die Formamidgruppen enthaltenden (Meth)-acrylsäureester durch Umsetzung von Alkanolaminen mit Ameisensäureestern und (Meth)-acrylsäurechlorid hergestellt werden.

Initiatoren im Rahmen der vorliegenden Erfindung sind Radikalbildner, die eine radikalische Polymerisation auslösen. Bevorzugt sind Fotoinitiatoren, die unter der Einwirkung von Licht, beispielsweise UV-Licht, sichtbarem Licht oder Laserlicht, eine radikalische Polymerisation auslösen.

Die sogenannten Fotopolymerisationsinitiatoren sind an sich bekannt (Houben-Weyl, Methoden der organischen Chemie, Band E20, Seite 80 ff, Georg Thieme Verlag Stuttgart 1987). Vorzugsweise handelt es sich um Mono- oder Dicarbonylverbindungen, wie Benzoin und dessen Derivate, insbesondere Benzoinmethylether, Benzil und Benzilderivate, beispielsweise 4,4-Oxidibenzil und andere Dicarbonylverbindungen wie Diacetyl, 2,3-Pentandion und α-Diketo-Derivate des Norbornans und substituierter Norbornane, Metallcarbonyle wie Pentacarbonylmangan oder Chinone wie 9,10-Phenanthrenchinon und Naphthochinon. Besonders bevorzugt ist Campherchinon.

Die erfindungsgemäßen Zubereitungen enthalten im allgemeinen 0,01 bis 2 Gew.-Teile, bevorzugt 0,1 bis 0,5 Gew.-Teile des Initiators, bezogen auf 1 Gew.-Teil des Formamidgruppen enthaltenden (Meth)-acrylsäureesters. Enthält eines der mit der erfindungsgemäßen Adhäsivkomponente im Kontakt stehenden Fügeteile bereits einen Initiator der beschriebenen Art, kann auf den Initiator in der Adhäsivkomponente auch ganz verzichtet werden.

Die Lösungsmittel im Rahmen der vorliegenden Erfindung sollen die Komponente lösen und sollen, durch die Anwendung bedingt, untoxisch sein. Bevorzugt seien Wasser und flüchtige organische Lösungsmittel wie Methanol, Ethanol, Propanol, Isopropanol, Aceton, Methylethylketon, Essigsäuremethyl- oder -ethylester genannt.

Im allgemeinen setzt man 10 bis 1000 Gew.-Teile, bevorzugt 50 bis 300 Gew.-Teile des Lösungsmittels, bezogen auf den Formamidgruppen enthaltenden (Meth)-acrylsäureester ein.

Es kann vorteilhaft sein, den erfindungsgemäßen Zubereitungen Coaktivatoren zuzusetzen, die die Polymerisationsreaktion beschleunigen. Bekannte Beschleuniger sind beispielsweise Amine wie p-Toluidin, Dimethyl-p-toluidin, Trialkylamine wie Trihexylamin, Polyamine wie N,N,N′,N′-Tetraalkylalkylendiamin, Barbitursäure und Dialkylbarbitursäure.

Die Coaktivatoren werden im allgemeinen in einer Menge von 0,02 bis 4 Gew.-%, bevorzugt 0,2 bis 1 Gew.-%, bezogen auf die Menge an polymerisierbaren Verbindungen eingesetzt.

Als weitere Komponente können die erfindungsgemäßen Zusammensetzungen Carbonylverbindungen enthalten.

Carbonylverbindungen im Rahmen der vorliegenden Erfindung sind Aldehyde und Ketone, die 1 bis 20, bevorzugt 1 bis 10, und besonders bevorzugt 2 bis 6 Kohlenstoffatome, enthalten. Die Carbonylfunktion kann an einem aliphatischen, aromatischen und heterocyclischen Molekülteil gebunden sein.

Als Aldehyde seien aliphatische Mono- oder Dialdehyde genannt. Bevorzugt wird Formaldehyd, Acetaldehyd, Propionaldehyde, 2-Methylpropionaldehyd, Butyraldehyd, Benzaldehyd, Vanillin, Furfurol, Anisaldehyd, Salicylaldehyd, Glyoxal, Glutardialdehyd und Phthaldialdehyd. Besonders bevorzugt ist der Glutardialdehyd.

Als Ketone seien besonders aliphatische Mono- und Diketone genannt. Bevorzugt sind Butanon, Aceton, Cyclooctanon, Cycloheptanon, Cyclohexanon, Cyclopentanon, Acetophenon, Benzophenon, 1-Phenyl-2-propanon, 1,3-Diphenyl-2-propanon, Acetylaceton, 1,2-Cyclohexandion, 1,2-Cyclopentandion und Campherchinon. Besonders bevorzugt ist Cyclopentanon.

Im allgemeinen setzt man 1 bis 1000 Gew.-Teile, bevorzugt 5 bis 50 Gew.-Teile der Carbonylverbindungen, bezogen auf den Formamidgruppen enthaltenden (Meth)-acrylsäureester, ein.

Als weitere Komponente können die erfindungsgemäßen Zusammensetzungen (Meth)-acrylsäureester enthalten, die Vernetzungen ausbilden können. (Meth)-acrylsäureester, die Vernetzungen ausbilden können, enthalten im allgemeinen 2 oder mehr polymerisierbare aktive Gruppen im Molekül. Bevorzugt seien Ester der (Meth)-acrylsäure mit 2- bis 5-wertigen Alkoholen mit 2 bis 30 Kohlenstoffatomen genannt. Besonders bevorzugt werden Alkoxy(meth)-acrylate und Urethangruppen enthaltende (Meth)acrylate.

Beispielsweise seien (Meth)-acrylsäureester der Formel 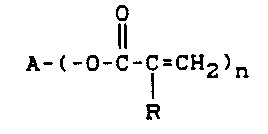
in der
- A: einen geradkettigen, verzweigten, cyclischen, aliphatischen, aromatischen oder gemischt aliphatischaromatischen Rest mit 2 bis 25 C-Atomen, der durch -O-, NH- oder O-CO-NH-Brücken unterbrochen und mit Hydroxy, Oxy, Carboxy, Amino oder Halogen substituiert sein kann,
bedeutet,
- R: H oder Methyl bedeutet und
- n: für eine ganze Zahl von 2 bis 8, vorzugsweise 2 bis 4, steht,
genannt.

Vorzugsweise seien Verbindungen der folgenden Formeln genannt: 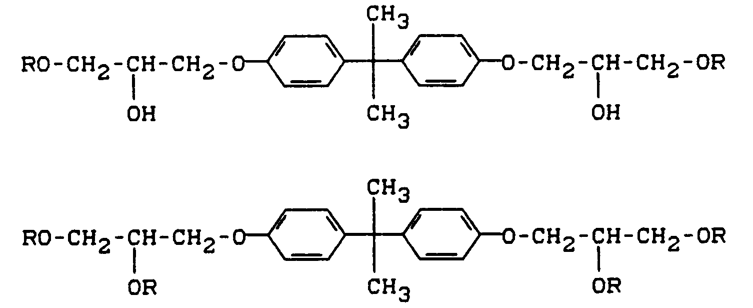 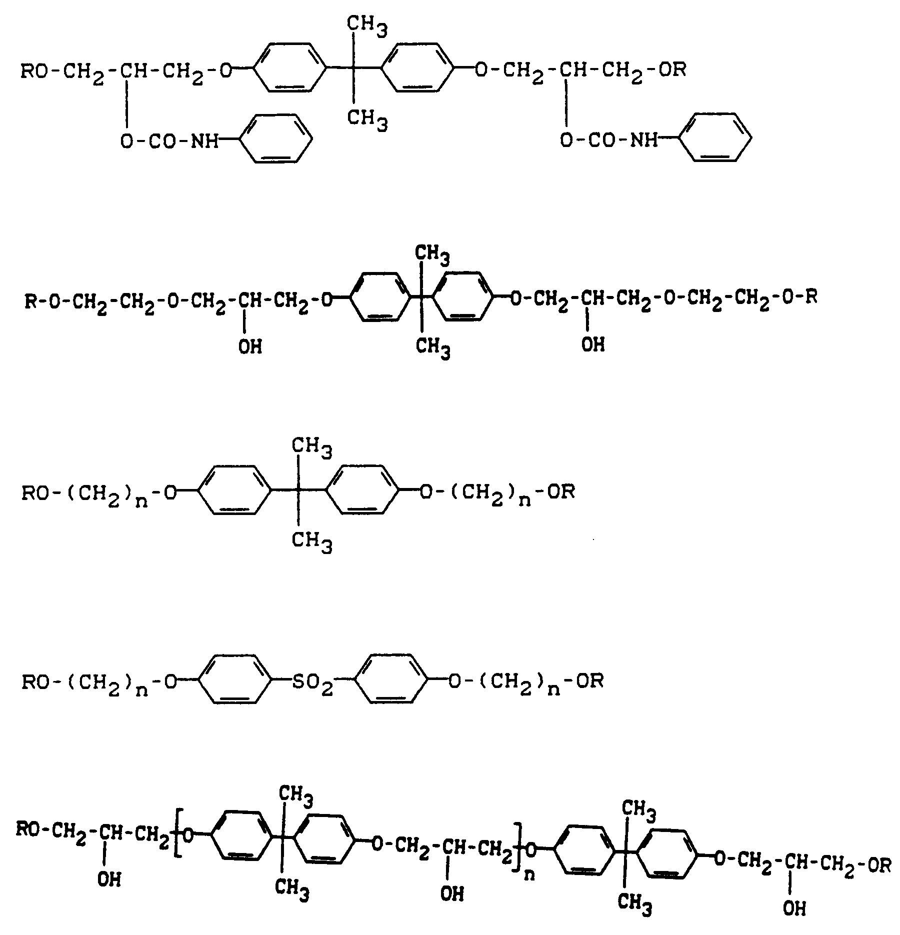 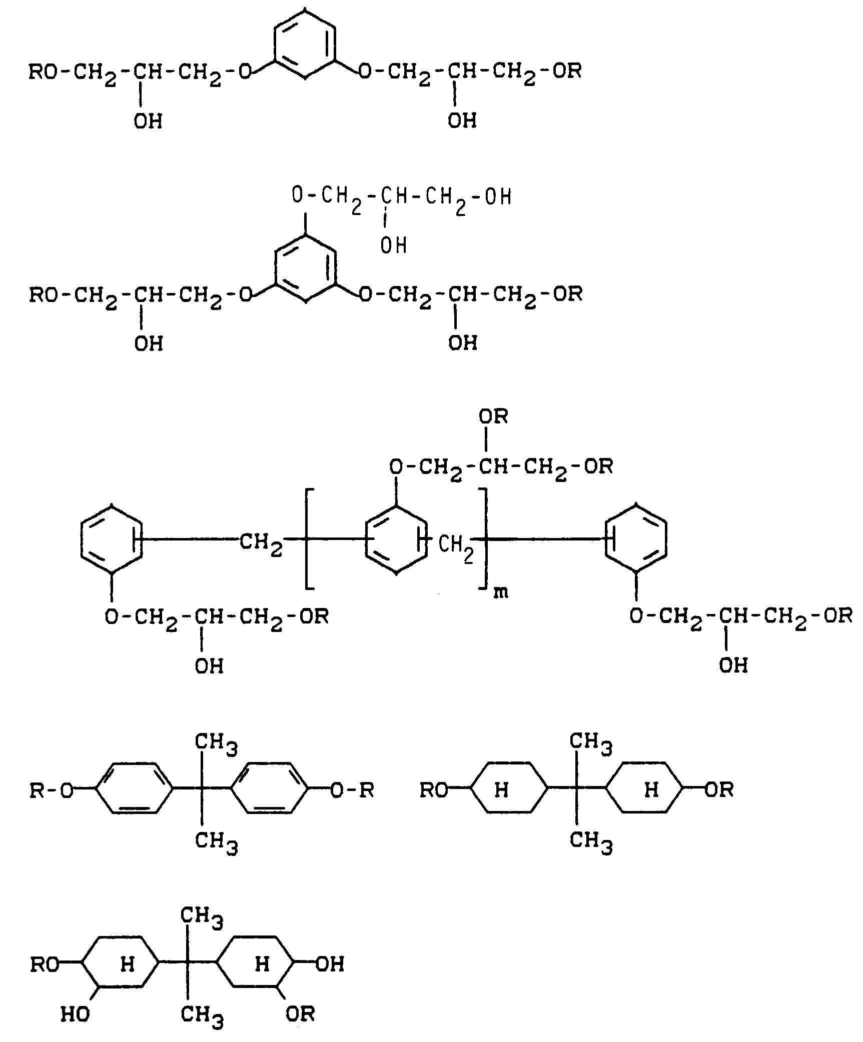 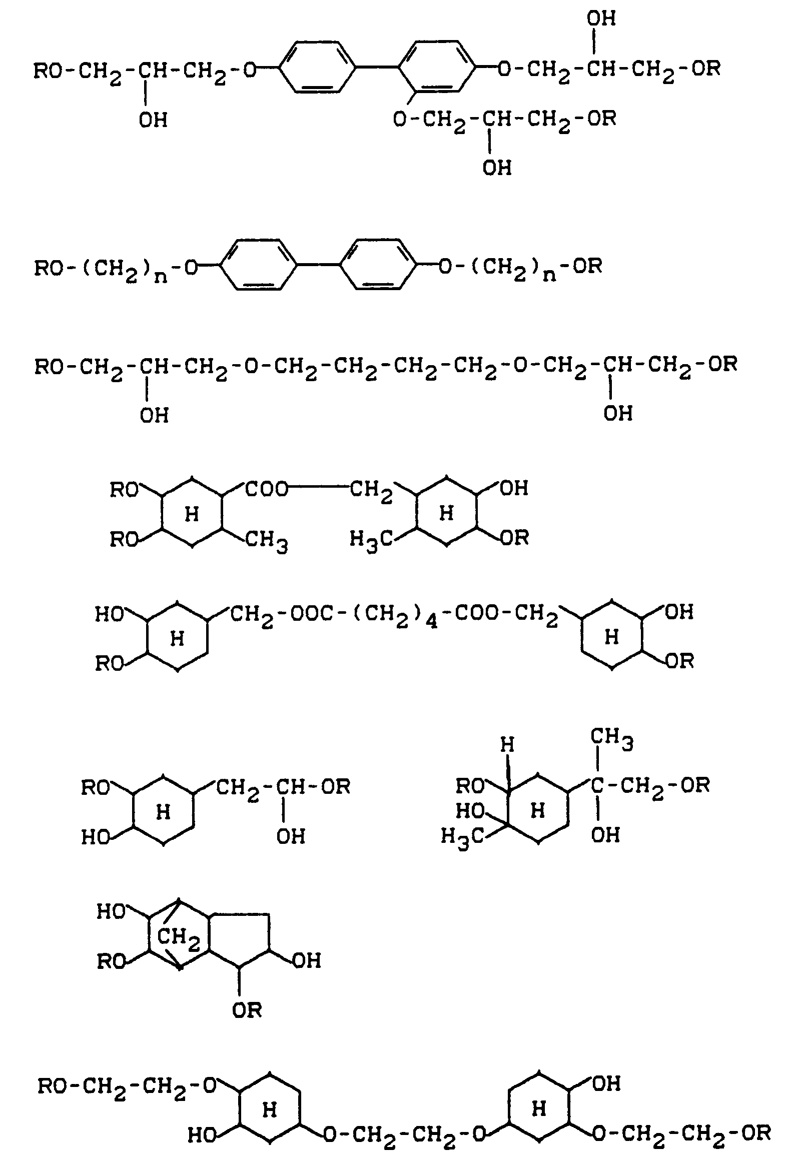 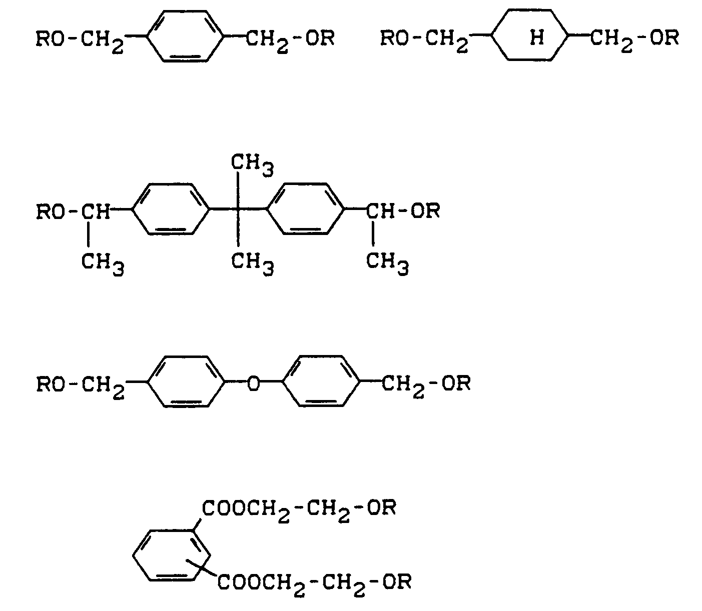
in der ortho-, meta- oder para-Form

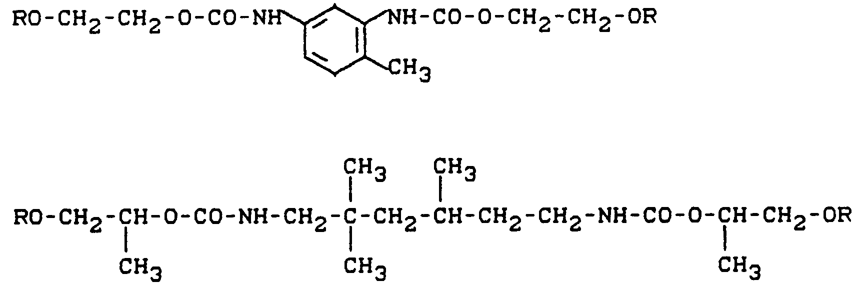

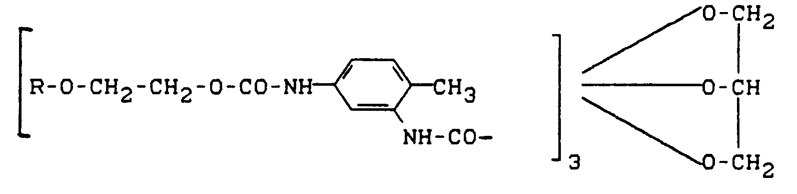

R-O-(CH₂)ₙ-O-R

R-O-(CH₂CH₂O)ₘ-R

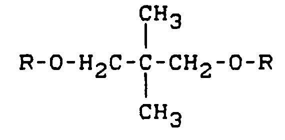

worin
- R: für 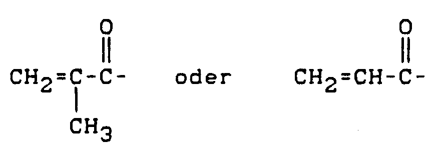 steht,
- n: eine Zahl von 1 bis 4 und
- m: eine Zahl von 0 bis 5 bedeutet.

Außerdem seien Derivate des Tricyclodecans (EP-A 00 23 686) und Umsetzungsprodukte aus Polyolen, Diisocyanaten und Hydroxyalkylmethacrylaten (DE-A 37 03 120, DE-A 37 03 080 und DE-A 37 03 130) genannt. Beispielsweise seien die folgenden Monomeren genannt: 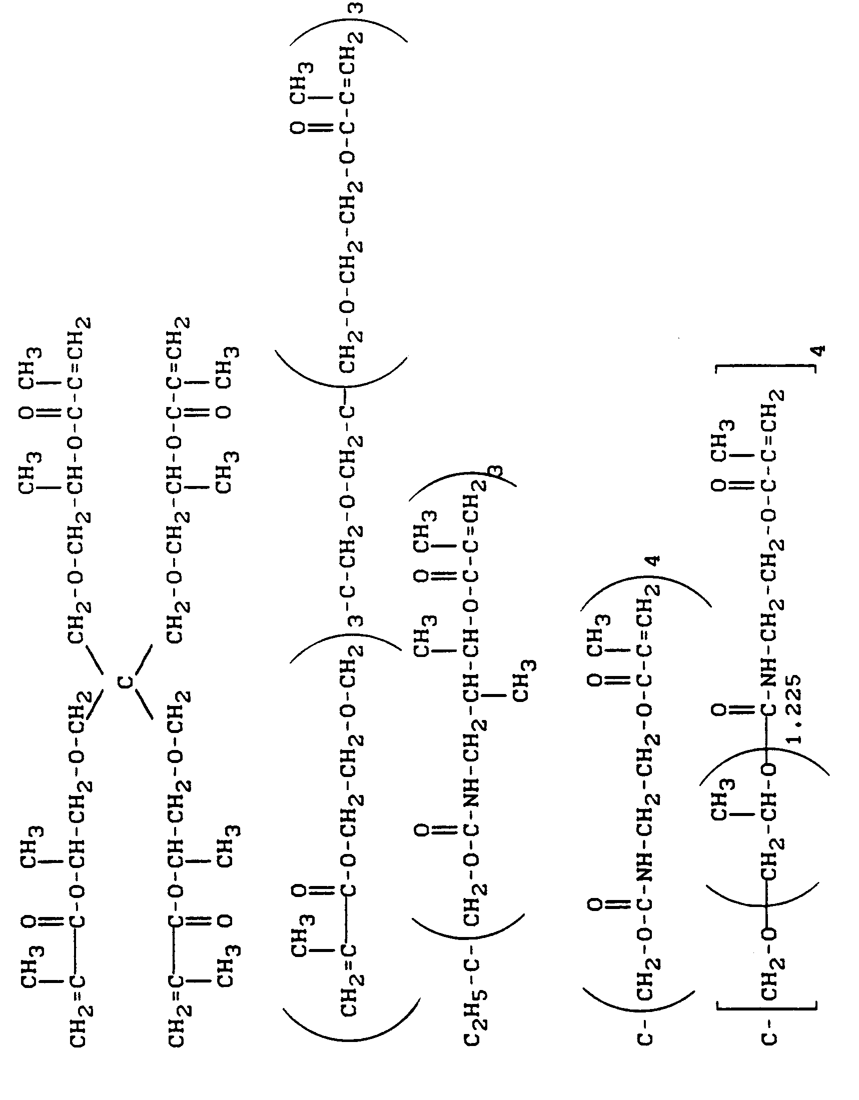 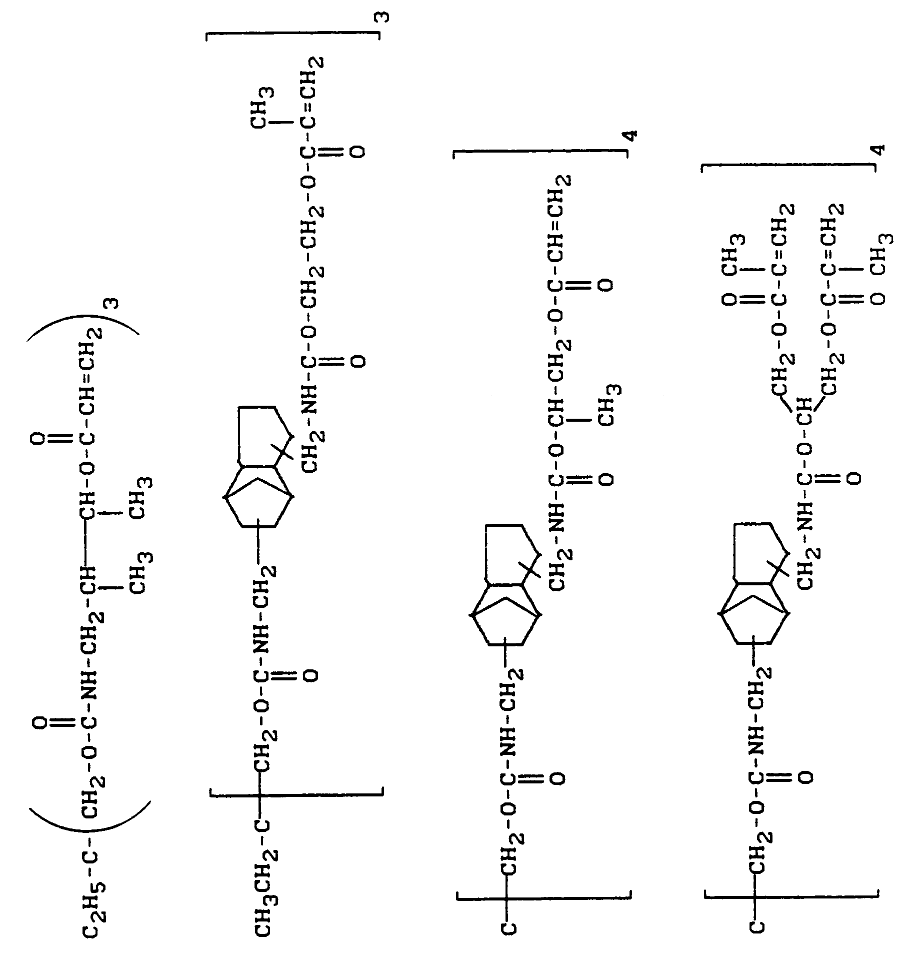 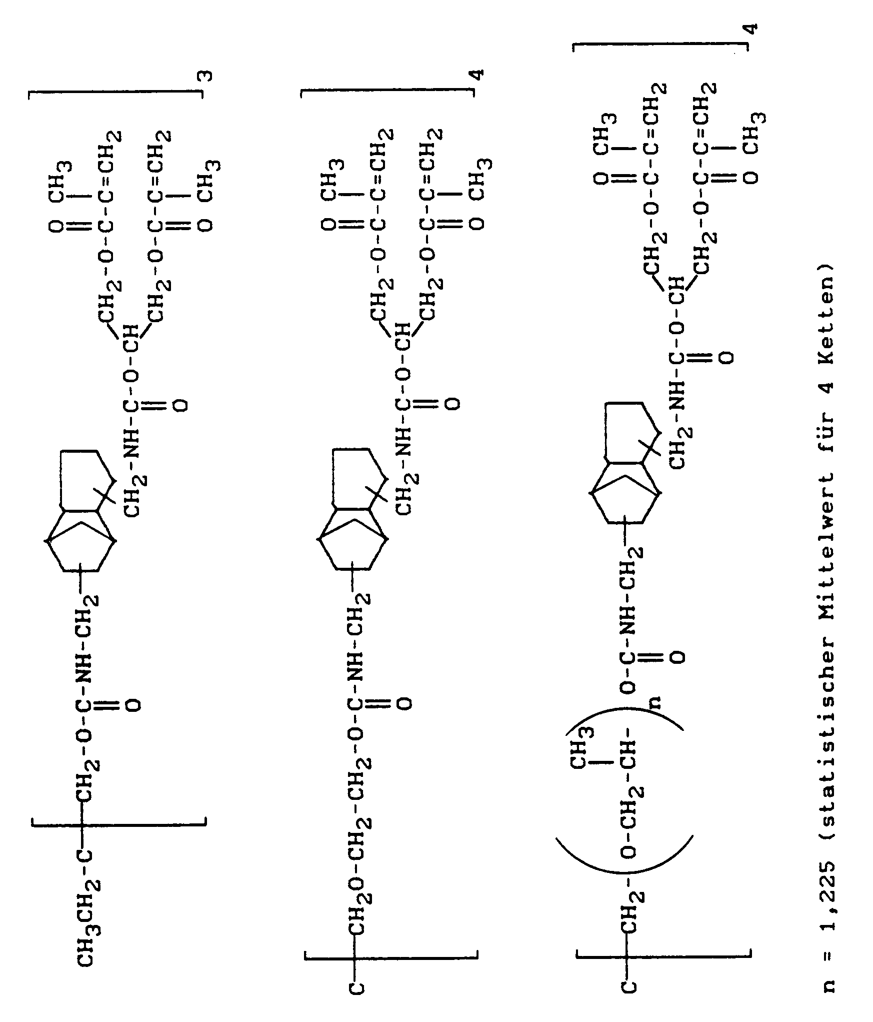 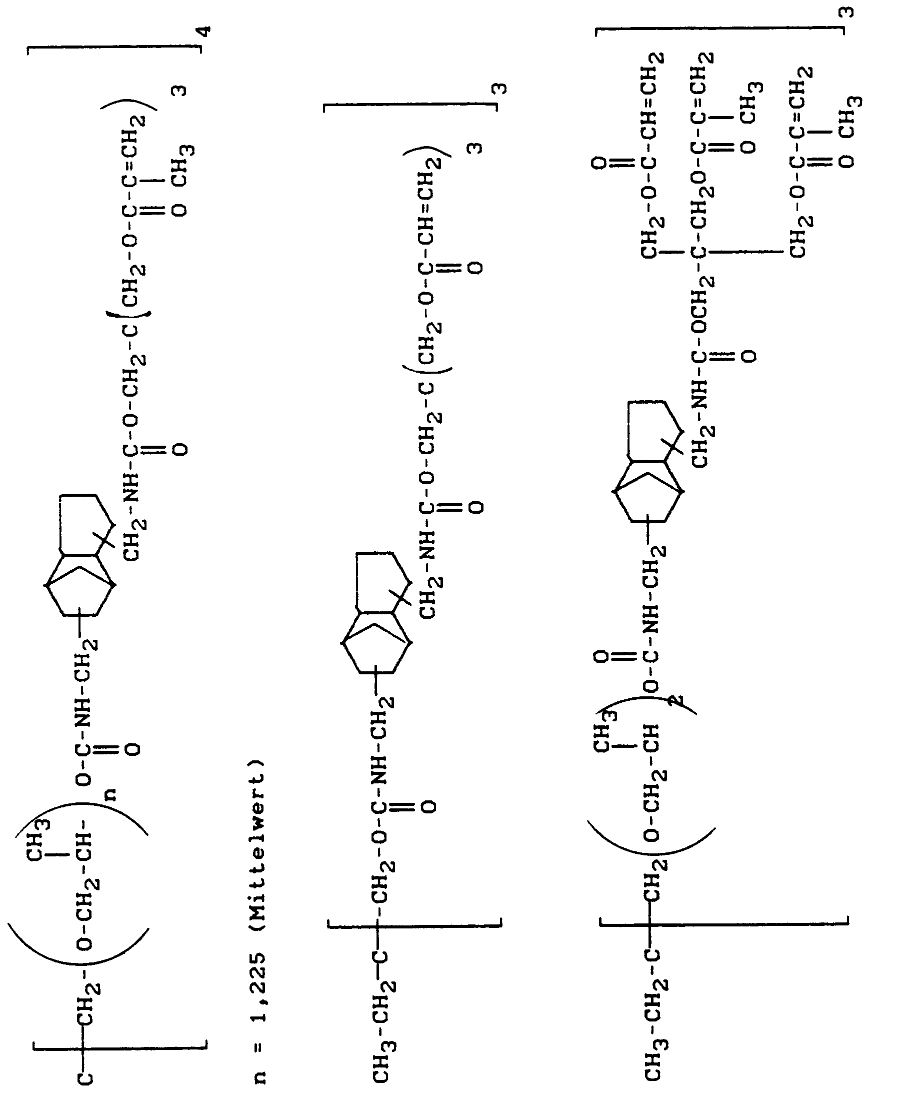 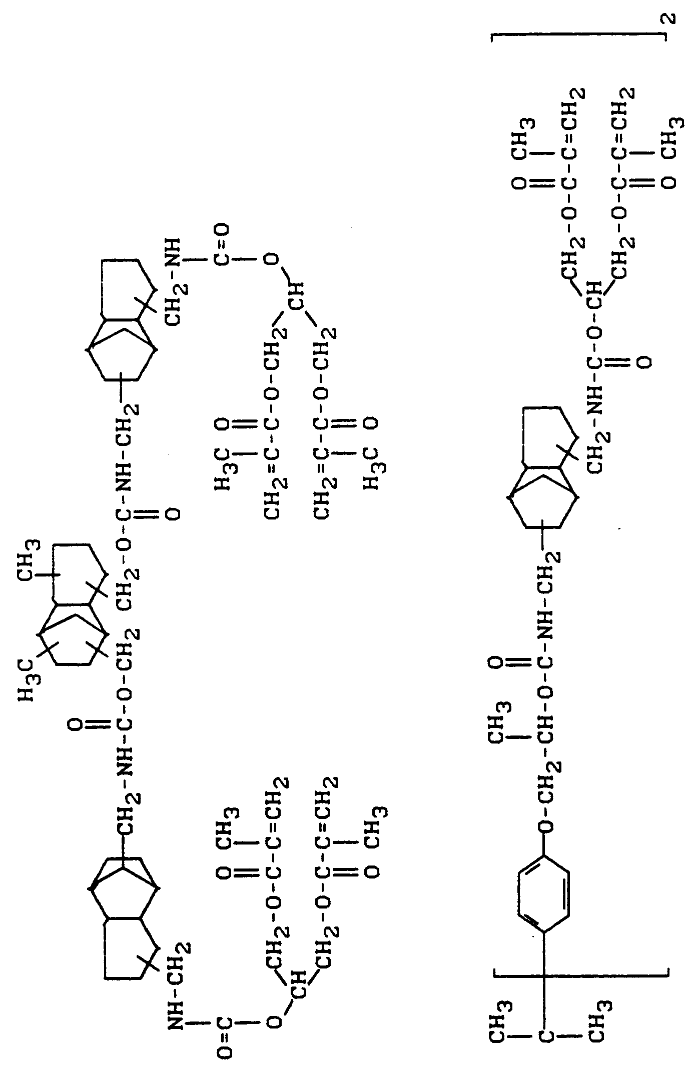 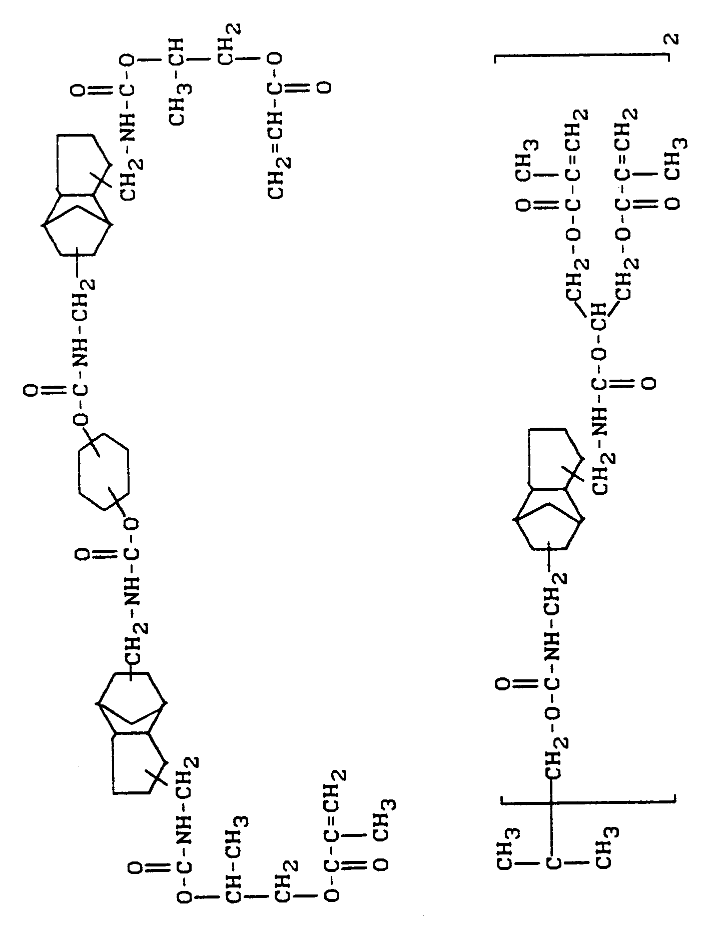 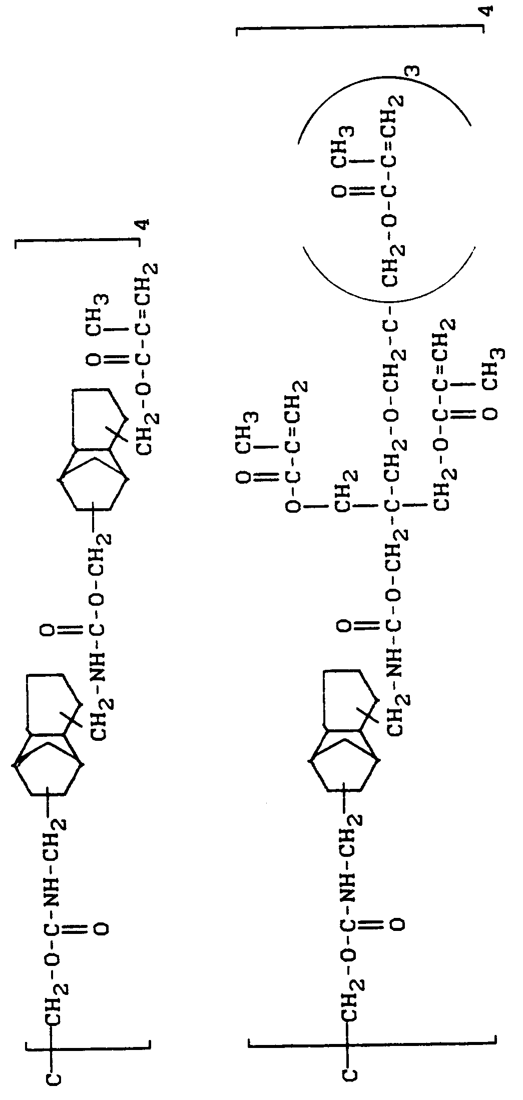 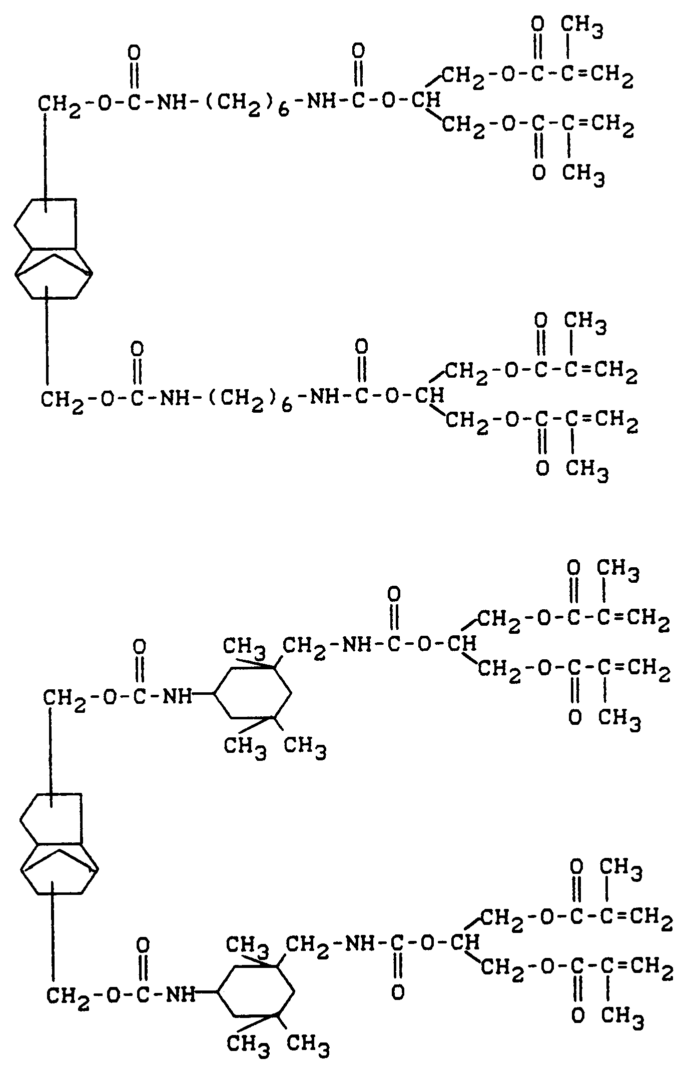 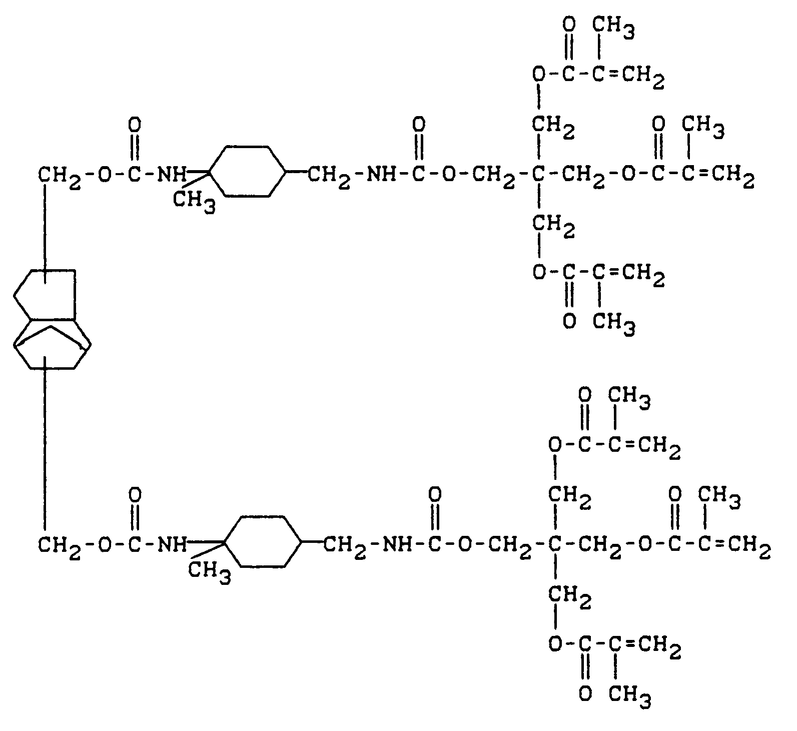 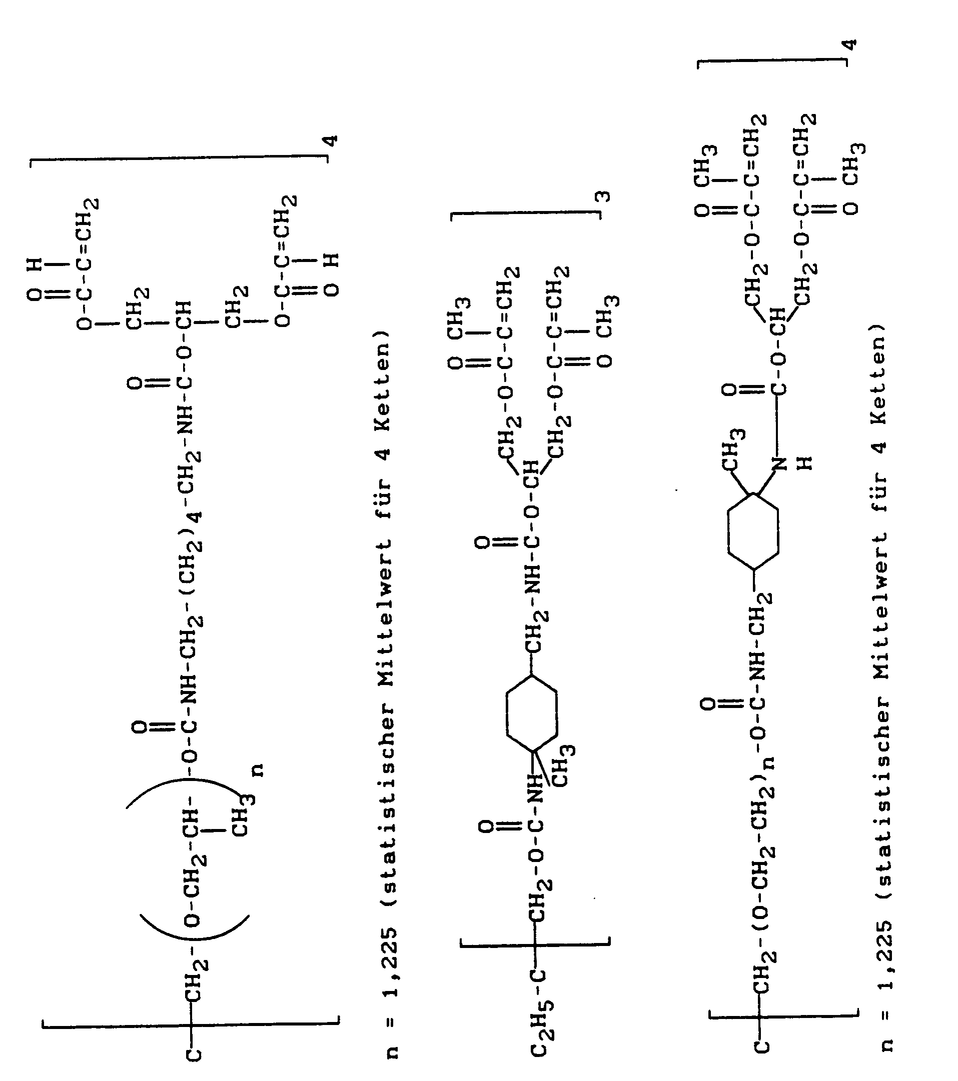 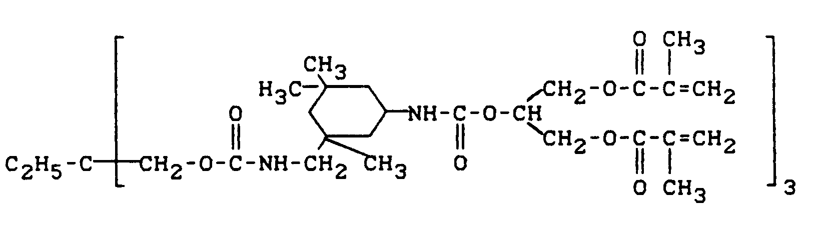
Besonders bevorzugt als Monomer wird das sogenannte Bis-GMA der Formel

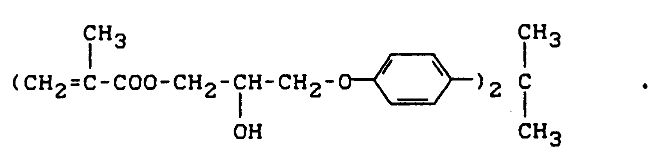

Selbstverständlich ist es möglich, Mischungen der verschiedenen (Meth)-Acrylsäureester, die Vernetzungen ausbilden können, einzusetzen. Beispielsweise seien Mischungen von 20 bis 70 Gew.-Teilen Bis-GMA und 30 bis 80 Gew.-Teilen Triethylenglykoldimethacrylat genannt.

Die erfindungsgemäßen Zubereitungen enthalten im allgemeinen 5 bis 80 Gew.-Teile, bevorzugt 10 bis 60 Gew.-Teile Carboxylverbindungen, bezogen auf die Formamidgruppen enthaltenden (Meth)-acrylsäureester.

Als weitere Komponente können die erfindungsgemäßen Zusammensetzungen Füllstoffe enthalten. Als Füllstoffe werden feine Pulver bevorzugt, die einen Teilchendurchmesser im Bereich von 0,1 bis 100 µm (gegebenenfalls auch in einer polydispersen Verteilung) haben. Füllstoffe können im Dentalbereich übliche Füllstoffe (R. S. Baratz, J. Biomat. Applications, Vol 1, 1987, S 316 ff) wie anorganische Gläser, Siliziumdioxid-, Aluminiumoxid-oder Quarzpulver sein.

Durch einen Anteil an Füllstoffen in den erfindungsgemäßen Zubereitungen entstehen Klebezemente, die besonders zur Befestigung von Brücken-, Kronen- und anderen Verblendmaterialen geeignet sind.

Der Anteil des Füllstoffs beträgt im allgemeinen 20 bis 80 Gew.-Teile, bevorzugt 40 bis 70 Gew.-Teile, bezogen auf die gesamte Zubereitung.

Die Adhäsivkomponenten gemäß dieser Erfindung können weiterhin bis zu 10 Gew.-Teilen üblicher Zusätze wie Stabilisatoren, Inhibitoren, Lichtschutzmittel, Farbstoffe, Pigmente oder Fluoreszenzstoffe enthalten.

Die erfindungsgemäßen Zubereitungen können hergestellt werden, indem man den Formamidgruppen enthaltenden (Meth)-acrylsäureester und den Initiator und gegebenenfalls die anderen Komponenten durch kräftiges Rühren mischt.

Die Zubereitungen können auch lösungsmittelfrei vorliegen.

Die erfindungsgemäßen Zubereitungen können als Adhäsivkomponente zur Behandlung kollagenhaltiger Materialien verwendet werden.

In einer besonderen Ausführungsform konditioniert man das kollagenhaltige Material vor der Behandlung mit der erfindungsgemäßen Zubereitung mit einer Flüssigkeit mit einem pH-Wert im Bereich von 0,1 bis 3,5.

Diese enthält im allgemeinen Säuren mit einem pKₛ-Wert kleiner als 5 und gegebenenfalls eine amphotere Aminoverbindung mit einem pKₛ-Wert im Bereich von 9,0 bis 10,6 und einem pK_{B}-Wert im Bereich von 11,5 bis 12,5. Folgende Säuren können z.B. in der Konditionierflüssigkeit enthalten sein:
Phosphorsäure, Salpetersäure, Brenztraubensäure, Zitronensäure, Oxalsäure, Ethylendiamintetraessigsäure, Essigsäure, Weinsäure, Äpfelsäure, Maleinsäure.

Als amphotere Aminoverbindungen seien vorzugsweise Verbindungen der Formel 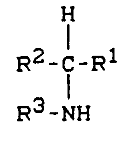
in der
- R¹: für eine Carboxylgruppe steht,
- R²: Wasserstoff, gegebenenfalls durch Hydroxy, Thio, Methylthio, Carboxy, Amino, Phenyl, Hydroxy-phenyl oder die Gruppen 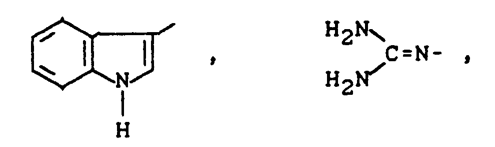 substituierter Niederalkylrest,
- R³: Wasserstoff oder Phenyl bedeutet und
wobei die Reste R¹ und R³ durch einen Propylrest verbunden sein können, oder
in der
- R¹: für Wasserstoff steht,
- R²: für die Gruppe

-A-NH₃X ,

in der
- A: für einen zweibindigen Alkylenrest mit 1 bis 6 Kohlenstoffatomen und
- X: für Halogen steht,
bedeutet und
- R³: Wasserstoff bedeutet,
genannt.

Beispielsweise seien die folgenden amphoteren Aminoverbindungen genannt: Glycin, Serin, Threonin, Cystein, Thyrosin, Asparagin, Glutamin, Alanin, Valin, Leucin, Isoleucin, Prolin, Methionin, Phenylalanin, Tryptophan, Lysin, Arginin, Histidin, N-Phenylglycin, Ethylendiaminhydrochlorid, Ethylendiaminhydrobromid, Propylendiaminhydrochlorid, Propylendiaminhydrobromid, Butylendiaminhydrochlorid, Butylendiaminhydrobromid, Leucinhydrochlorid und Histidinhydrochlorid.

Weiterhin kann die Konditionierflüssigkeit Stoffe aus der Gruppe der Polyethylenglykole und Metallhydroxide enthalten. Insbesondere können die oben aufgeführten mehrbasigen Säuren auch als teilweise Metallsalze eingesetzt werden solange freie Säurefunktionen verbleiben.

Konditionierflüssigkeiten, die mindestens eine der Säuren aus der Gruppe der Brenztraubensäure, Ethylendiamintetraessigsäure und Zitronensäure sowie gegebenenfalls eine amphotere Aminoverbindung aus der Gruppe des Glycins, N-Phenylglycins und Prolins enthalten, sind besonders bevorzugt.

Die Anwendung der erfindungsgemäßen Zubereitungen kann beispielsweise wie folgt durchgeführt werden:
Bei einer Zahnreparatur zum Beispiel trägt man beispielsweise nach einer mechanischen Reinigung des kollagenhaltigen Zahnmaterials zuerst die Konditionierflüssigkeit mit etwas Watte auf, läßt eine kurze Zeit (beispielsweise 60 Sekunden) einwirken, spült das Zahnmaterial mit Wasser und trocknet es im Luftstrom. Dann trägt man die erfindungsgemäße Zubereitung beispielsweise mit einem kleinen Pinsel in einer dünnen Schicht auf und trocknet im Luftstrom. Nach der erfindungsgemäßen Behandlung trägt man die eigentliche Füllmasse, beispielsweise im Dentalbereich übliche Kunststoffüllungsmassen (K. Eichner, "Zahnärztliche Werkstoffe und ihre Verarbeitung", Bd. 2, S. 135 ff, Hüthig Verlag, 5. Aufl. 1985) auf.

In ähnlicher Weise können die erfindungsgemäßen Zubereitungen zur Befestigung von Zahn reparature materialien, wie Kronen, Brücken und ähnlichen Hilfsmitteln verwendet werden.

### Beispiele 1 bis 9 (Herstellung)

Die erfindungsgemäßen Klebemittel werden durch intensives Vermischen der in folgenden Beispielen aufgeführten Bestandteile erzeugt.

### Beispiel 1:

130 g Wasser
110 g Methacrylsäure-2-formamidoethylester
300 mg Camperchinon

### Beispiel 2:

130 g Wasser
110 g Methacrylsäure-3-formamidopropylester
300 mg Campherchinon

### Beispiel 3:

100 g Wasser
100 g Methacrylsäure-3-formamidopropylester
43 g 25 Gew.-%ige wäßrige Glutardialdehyd-Lösung
300 mg Campherchinon

### Beispiel 4:

100 g Wasser
100 g Methacrylsäure-3-formamidopropylester
43 g 25 gew.-%ige wäßrige Glutardialdehyd-Lösung
76 g 2-Hydroxyethylmethacrylat
300 mg Campherchinon

### Beispiel 5:

130 g Wasser
110 g Methacrylsäure-5-formamidopentylester
300 mg Campherchinon

### Beispiel 6:

100 g Wasser
100 g Methacrylsäure-5-formamidopentylester
43 g 25 gew.-%ige wäßrige Glutardialdehyd-Lösung
300 mg Campherchinon

### Beispiel 7:

100 g Wasser
100 g Methacrylsäure-2-(N-methylformamido)-ethylester
43 g 25 gew.-%ige wäßrige Glutardialdehyd-Lösung
300 mg Campherchinon

### Beispiel 8:

260 g Wasser
110 g Methacrylsäure-2-(N-methylformamido)-ethylester
300 mg Campherchinon

### Beispiel 9:

100 g Wasser
110 g Methacrylsäure-2-(N-2-hydroxyethylformamido)ethylester
43 g 25 gew.-%ige wäßrige Glutardialdehyd-Lösung
300 mg Campherchinon

### Beispiel 10:

130 g Wasser
120 g Methacrylsäure-2-(N-2-hydroxyethylformamido)ethylester
300 mg Campherchinon
120 mg Isobutyraldehyd
120 mg 2,6-Di-tert.-butyl-4-methylphenol
120 mg Hydrochinonmonomethylether

### Beispiel 11:

100 g Wasser
80 g Acrylsäure-2-Formamidoethylester
44 g 25 gew.-%ige wäßrige Glutardialdehyd-Lösung
300 mg Campherchinon

### Beispiel 12: (Anwendung)

Die Eignung der Klebemittel entsprechend der Beispiele 1 bis 11 wird geprüft, indem die Bindungsfestigkeit der lichtaktivierten Kunststoffüllungsmasse auf Basis mehrfunktioneller Methacrylsäureester und Bariumaluminosilikat Lumifor® auf Dentin bestimmt wird, das nacheinander mit der Konditionierflüssigkeit (bestehend aus 81,2 g Wasser, 1,7 g Natriumhydroxid und 17 g Ethylendiamintetraessigsäure-Dinatrium-Dihydrat: 60 Sekunden Einwirkzeit, Wasserspülung, Lufttrocknung), dem Klebemittel (60 Sekunden Einwirkzeit, Lufttrocknung) und einem Verschlußmaterial auf Basis polyfunktioneller Methacrylsäureester (Bayer Resin L®) (Auftragen und im Luftstrom dünn verteilen) vorbehandelt worden ist.

Für den Test werden herausgezogene und im feuchten Zustand aufbewahrte Menschenzähne benutzt. Die Zähne werden durch Guß in Epoxidharz eingelagert; durch Nachschleifen wird eine glatte Dentinoberfläche erzeugt. Das abschließende Schleifen erfolgt mit Carbonpapier 1000.

Zum Herstellung eines Probekörpers zum Messen der Bindungsstärke wird eine zylindrische, gespaltete Teflonform auf die wie vorstehend beschrieben behandelte Dentinoberfläche gespannt (Scand. J. Dent. Res. 88, 348-351 (1980)). Als Füllmasse wird ein handelsübliches Kunststoffüllungsmaterial eingefüllt. Ein in einem Loch in einer Bohrerhaltung eingespannter Rundbohrer Nr. 016 wird an der Teflonform befestigt und von oben in die noch im Härteprozeß befindliche Materialschicht gepreßt.

Die gesamte Anordnung wird 10 Minuten lang bei Zimmertemperatur (25°C) ungestört stehengelassen, wonach die Bohrerhalterung und die Teflonform abgenommen und die Probe unter Wasser mit einer Temperatur von 23°C abgesetzt wird. Nach 15 Minuten wird die Probe mit dem Bohrer in eine Instron-Zugversuchsapparatur (Scand. J. Dent. Res. 88, 348-351 (1980)) montiert; mit einer Geschwindigkeit von 1 mm/Min. wird eine Zugfestigkeitsmessung durchgeführt. Die Zugfestigkeit errechnet sich aus der Division der beim Bruch der Füllung angelegten Belastung mit dem Querschnittsareal in der Bruchfläche des Probekörpers. Es wurden jeweils 5 Messungen an Probekörpern durchgeführt.

Die Ergebnisse sind in der folgenden Tabelle zusammengefaßt:

| Zubereitung nach Beispiel Nr. | Zugbindungsfestigkeit [N/mm²] |
|---|---|
| 1 | 22.5 ± 1.1 |
| 2 | 15.0 ± 2.5 |
| 3 | 19.3 ± 2.3 |
| 4 | 19.4 ± 2.1 |
| 5 | 3.7 ± 1.0 |
| 6 | 6.8 ± 2.4 |
| 7 | 19.1 ± 2.0 |
| 8 | 20.0 ± 1.0 |
| 9 | 19.8 ± 2.3 |
| 10 | 15.2 ± 2.2 |
| 11 | 21.7 ± 3.8 |

## Patentansprüche (Patentansprüche für folgende(n) Vertragsstaat(en): AT, BE, CH, DE, FR, GB, IT, LI, LU, NL, SE)

1. Adhäsiv Zubereitung, enthaltend Formamidgruppen enthaltende (Meth)-acrylsäureester der Formel 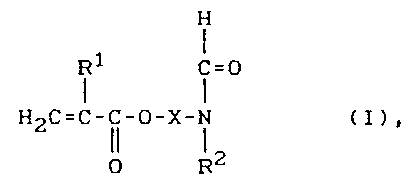 in der
R¹ Wasserstoff oder Methyl bedeutet,
R² Wasserstoff, gegebenenfalls durch Hydroxy, Carboxy, Halogen oder Amino der Formel 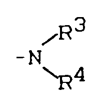 in der
R³ und R⁴ gleich oder verschieden sind und Wasserstoff oder Niederalkyl bedeuten,
substituiertes Alkyl (C₁ bis C₁₂), Aryl (C₆ bis C₁₂) oder Aralkyl (C₇ bis C₁₄) bedeutet, und
X ein zweiwertiger, gegebenenfalls durch Hydroxy, Carboxy, Halogen oder Amino der Formel 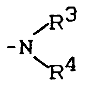 in der
R³ und R⁴ die obengenannte Bedeutung haben,
substituierter aliphatischer (C₁ bis C₂₄) und/oder cycloaliphatischer (C₅ bis C₈) und/oder aromatischer (C₆ bis C₁₂) Rest, der gegebenenfalls eine oder mehrere Sauerstoff-, Schwefel- und/oder -NR³-Brücken enthalten kann,
wobei
R³ die obengenannte Bedeutung hat,
bedeutet,
und gegebenenfalls Initiatoren
zur Anwendung in einem Verfahren zur therapeutischen Behandlung kollagenhaltiger menschlicher und tierischer interzellularer Stützsubstanzen.

2. Zubereitung nach Anspruch 1, enthaltend Formamidgruppen enthaltende (Meth)-acrylsäureester,
worin
R¹ Wasserstoff oder Methyl bedeutet,
R² Wasserstoff, gegebenenfalls durch Hydroxy, Carboxy, Fluor, Chlor oder Amino substituiertes Alkyl (C₁ bis C₆), Phenyl, Tolyl, Benzyl oder Methyl substituiertes Benzyl bedeutet, und
X einen zweiwertigen, gegebenenfalls durch Hydroxy, Carboxy, Fluor, Chlor oder Amino substituierten, aliphatischen (C₁ bis C₂₀) und/oder cycloaliphatischen (C₅ und C₆) und/oder aromatischen (C₆ bis C₁₂)-Rest, der gegebenenfalls ein bis zehn Sauerstoff- oder -NR³-Brücken enthalten kann,
wobei R³ die obengenannte Bedeutung hat,
bedeutet.

3. Zubereitung nach den Ansprüchen 1 und 2, enthaltend Formamidgruppen enthaltende (Meth)-acrylsäureester,
worin
R¹ Wasserstoff oder Methyl bedeutet,
R² Wasserstoff, gegebenenfalls durch Hydroxy, Carboxy, Fluor, Chlor oder Amino substituiertes Alkyl (C₁ bis C₆), Phenyl oder Tolyl bedeutet, und
X einen zweiwertigen, gegebenenfalls durch Hydroxy, Carboxy, Fluor, Chlor oder Amino substituierten, aliphatischen (C₁ bis C₈)- und/oder cycloaliphatischen (C₅ und C₆)- und/oder Phenylen-Rest, der gegebenenfalls ein bis vier Sauerstoffbrücken enthalten kann, bedeutet.

4. Zubereitung nach den Ansprüchen 1 bis 3, dadurch gekennzeichnet, daß als Initiator ein Radikalbildner aus der Reihe der Mono- oder Dicarbonylverbindungen eingesetzt wird.

5. Zubereitung nach den Ansprüchen 1 bis 4, dadurch gekennzeichnet, daß der Formamidgruppen enthaltende (Meth)-acrylsäureester und der Initiator in einem Lösungsmittel gelöst sind.

6. Zubereitung nach den Ansprüchen 1 bis 5, dadurch gekennzeichnet, daß ein Coaktivator zugesetzt wird.

7. Zubereitung nach den Ansprüchen 1 bis 6, dadurch gekennzeichnet, daß als weitere Komponente eine Carbonylverbindung zugesetzt wird.

8. Zubereitung nach den Ansprüchen 1 bis 7, dadurch gekennzeichnet, daß als weitere Komponente (Meth)acrylsäureester, der Vernetzungen ausbilden kann, zugesetzt wird.

9. Zubereitung nach den Ansprüchen 1 bis 8, dadurch gekennzeichnet, daß als weitere Komponente ein Füllstoff zugesetzt wird.

10. Verfahren zur Herstellung von Adhäsiv Zubereitungen zur Anwendung in einem Verfahren zur therapeutischen Behandlung kollagenhaltiger menschlicher und tierischer interzellularer Stützsubstanzen, dadurch gekennzeichnet, daß man den Formamidgruppen enthaltenden (Meth)-acrylsäureester der Formel 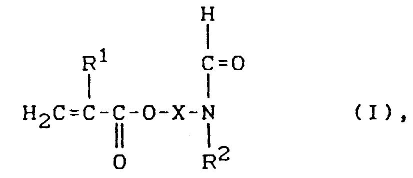 in der
R¹ Wasserstoff oder Methyl bedeutet,
R² Wasserstoff, gegebenenfalls durch Hydroxy, Carboxy, Halogen oder Amino der Formel 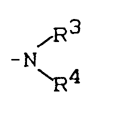 in der
R³ und R⁴ gleich oder verschieden sind und Wasserstoff oder Niederalkyl bedeuten,
substituiertes Alkyl (C₁ bis C₁₂), Aryl (C₆ bis C₁₂) oder Aralkyl (C₇ bis C₁₄) bedeutet, und
X ein zweiwertiger, gegebenenfalls durch Hydroxy, Carboxy, Halogen oder Amino der Formel 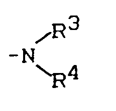 in der
R³ und R⁴ die obengenannte Bedeutung haben,
substituierter aliphatischer (C₁ bis C₂₄) und/oder cycloaliphatischer (C₅ bis C₈) und/oder aromatischer (C₆ bis C₁₂) Rest, der gegebenenfalls eine oder mehrere Sauerstoff-, Schwefel- und/oder -NR³-Brücken enthalten kann,
wobei
R³ die obengenannte Bedeutung hat,
bedeutet
mit den weiteren Komponenten der Zubereitung vermischt.

11. Verwendung von Zubereitungen enthaltend Formamidgruppen enthaltende (Meth)-acrylsäureester der Formel 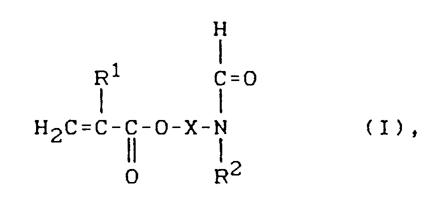 in der
R¹ Wasserstoff oder Methyl bedeutet,
R² Wasserstoff, gegebenenfalls durch Hydroxy, Carboxy, Halogen oder Amino der Formel 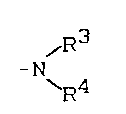 in der
R³ und R⁴ gleich oder verschieden sind und Wasserstoff oder Niederalkyl bedeuten,
substituiertes Alkyl (C₁ bis C₁₂), Aryl (C₆ bis C₁₂) oder Aralkyl (C₇ bis C₁₄) bedeutet, und
X ein zweiwertiger, gegebenenfalls durch Hydroxy, Carboxy, Halogen oder Amino der Formel 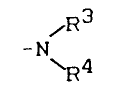 in der
R³ und R⁴ die obengenannte Bedeutung haben,
substituierter aliphatischer (C₁ bis C₂₄) und/oder cycloaliphatischer (C₅ bis C₈) und/oder aromatischer (C₆ bis C₁₂) Rest, der gegebenenfalls eine oder mehrere Sauerstoff-, Schwefel- und/oder -NR³-Brücken enthalten kann,
wobei
R³ die obengenannte Bedeutung hat,
bedeutet,
und gegebenenfalls einen Initiator zur Herstellung von Adhäsiv- Mitteln zur Anwendung in einem Verfahren zur therapeutischen Behandlung kollagenhaltiger, menschlicher und tierischer interzellularer Stützsubstanzen.

12. Verwendung nach Anspruch 11, nach einer Konditionierung des kollagenhaltigen Materials mit einer Flüssigkeit eines pH-Wertes von 0,1 bis 3,5.

13. Verwendung nach den Ansprüchen 11 und 12 als Klebemittel zur Befestigung von Zahnreparaturmaterialien am Zahn.

14. Verwendung nach Anspruch 11 als Klebemittel in Knochenzement.

## Patentansprüche (Patentansprüche für folgende(n) Vertragsstaat(en): ES, GR)

1. Verfahren zur Herstellung von Adhäsiv Zubereitungen zur Anwendung in einem Verfahren zur therapeutischen Behandlung kollagenhaltiger menschlicher und tierischer interzellularer Stützsubstanzen, dadurch gekennzeichnet, daß man den Formamidgruppen enthaltenden (Meth)-acrylsäureester der Formel 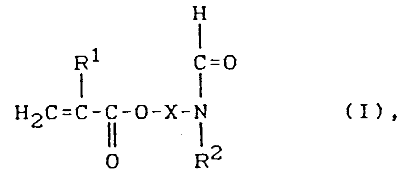 in der
R¹ Wasserstoff oder Methyl bedeutet,
R² Wasserstoff, gegebenenfalls durch Hydroxy, Carboxy, Halogen oder Amino der Formel 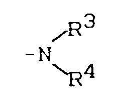 in der
R³ und R⁴ gleich oder verschieden sind und Wasserstoff oder Niederalkyl bedeuten,
substituiertes Alkyl (C₁ bis C₁₂), Aryl (C₆ bis C₁₂) oder Aralkyl (C₇ bis C₁₄) bedeutet, und
X ein zweiwertiger, gegebenenfalls durch Hydroxy, Carboxy, Halogen oder Amino der Formel 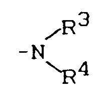 in der
R³ und R⁴ die obengenannte Bedeutung haben,
substituierter aliphatischer (C₁ bis C₂₄) und/oder cycloaliphatischer (C₅ bis C₈) und/oder aromatischer (C₆ bis C₁₂) Rest, der gegebenenfalls eine oder mehrere Sauerstoff-, Schwefel- und/oder -NR³-Brücken enthalten kann,
wobei
R³ die obengenannte Bedeutung hat,
bedeutet
mit der weiteren Komponenten der Zubereitung vermischt.

## Claims (Claims for the following Contracting State(s): AT, BE, CH, DE, FR, GB, IT, LI, LU, NL, SE)

1. Adhesive formulation which contains (meth)acrylic acid esters, containing formamide groups, of the formula 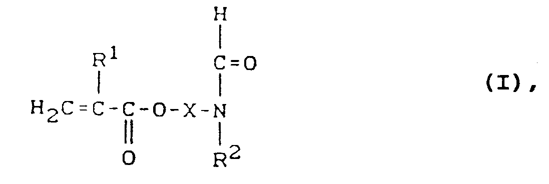 in which
R¹ denotes hydrogen or methyl,
R² denotes hydrogen, optionally hydroxy-, carboxy-, halogen- or amino-substituted (amino of the formula 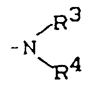 in which
R³ and R⁴ are identical or different and denote hydrogen or lower alkyl)
alkyl (C₁ to C₁₂), aryl (C₆ to C₁₂) or aralkyl (C₇ to C₁₄), and
X denotes a divalent, optionally hydroxy-, carboxy-, halogen- or amino-substituted (amino of the formula 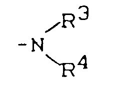 in which
R³ and R⁴ have the meaning given above),
aliphatic (C₁ to C₂₄) and/or cycloaliphatic (C₅ to C₈) and/or aromatic (C₆ to C₁₂) radical which can optionally contain one or more oxygen bridges, sulphur bridges and/or -NR³ bridges,
R³ having the meaning given above,
and optionally initiators,
for use in a process for the therapeutic treatment of collagen-containing human and animal intercellular supporting substances.

2. Formulation according to Claim 1, which contains (meth)acrylic acid esters, containing formamide groups,
wherein
R¹ denotes hydrogen or methyl,
R² denotes hydrogen, optionally hydroxy-, carboxy-, fluorine-, chlorine- or amino-substituted alkyl (C₁ to C₆), phenyl, tolyl, benzyl or methyl-substituted benzyl, and
X denotes a divalent, optionally hydroxy-, carboxy-fluorine-, chlorine- or amino-substituted aliphatic (C₁ to C₂₀) and/or cycloaliphatic (C₅ or C₆) and/or aromatic (C₆ to C₁₂) radical which can optionally contain one to ten oxygen bridges or -NR³ bridges,
R³ having the meaning given above.

3. Formulation according to Claims 1 and 2, which contains (meth) acrylic acid esters, containing formamide groups,
wherein
R¹ denotes hydrogen or methyl,
R² denotes hydrogen, optionally hydroxy-, carboxy-, fluorine-, chlorine- or amino-substituted alkyl (C₁ to C₆), phenyl or tolyl, and
X denotes a divalent, optionally hydroxy-, carboxy-, luorine-, chlorine- or amino-substituted aliphatic (C₁ to C₈) and/or cycloaliphatic (C₅ or C₆) and/or phenylene radical which can optionally contain one to four oxygen bridges.

4. Formulation according to Claims 1 to 3, characterized in that a free-radical former from the series of the monocarbonyl or dicarbonyl compounds is used as the initiator.

5. Formulation according to Claims 1 to 4, characterized in that the (meth)acrylic acid ester containing formamide groups and the initiator are dissolved in a solvent.

6. Formulation according to Claims 1 to 5, characterized in that a coactivator is added.

7. Formulation according to Claims 1 to 6, characterized in that a carbonyl compound is added as a further component.

8. Formulation according to Claims 1 to 7, characterized in that a (meth)acrylic acid ester, which can form crosslinks, is added as a further component.

9. Formulation according to Claims 1 to 8, characterized in that a filler is added as a further component.

10. Process for preparing adhesive formulations for use in a process for the therapeutic treatment of collagen-containing human and animal intercellular supporting substances characterized in that the (meth)acrylic acid ester, containing formamide groups, of the formula 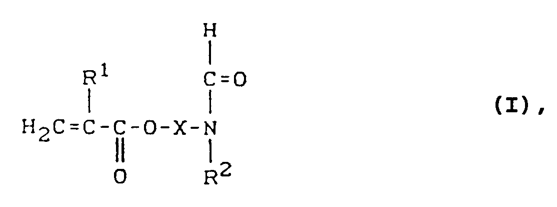 in which
R¹ denotes hydrogen or methyl,
R² denotes hydrogen, optionally hydroxy-, carboxy-, halogen- or amino-substituted (amino of the formula 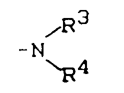 in which
R³ and R⁴ are identical or different and denote hydrogen or lower alkyl)
alkyl (C₁ to C₁₂), aryl (C₆ to C₁₂) or aralkyl (C₇ to C₁₄), and
X denotes a divalent, optionally hydroxy-, carboxy-, halogen- or amino-substituted (amino of the formula 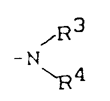 in which
R³ and R⁴ have the meaning given above)
aliphatic (C₁ to C₂₄) and/or cycloaliphatic (C₅ to C₈) and/or aromatic (C₆ to C₁₂) radical which can optionally contain one or more oxygen bridges, sulphur bridges and/or -NR³ bridges,
R³ having the meaning given above,
is mixed, with the further components of the formulation.

11. Use of formulations which contain (meth)acrylic acid eaters, containing formamide groups, of the formula 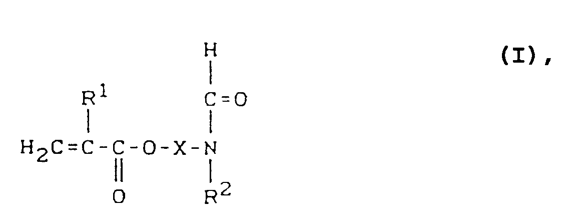 in which
R¹ denotes hydrogen or methyl,
R² denotes hydrogen, optionally hydroxy-, carboxy-, halogen- or amino-substituted (amino of the formula 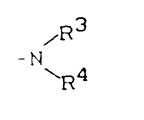 in which
R³ and R⁴ are identical or different and denote hydrogen or lower alkyl)
alkyl (C₁ to C₁₂), aryl (C₆ to C₁₂) or aralkyl (C₇ to C₁₄), and
X denotes a divalent, optionally hydroxy-, carboxy-, halogen- or amino-substituted (amino of the formula 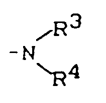 in which
R³ and R⁴ have the meaning given above)
aliphatic (C₁ to C₂₄) and/or cycloaliphatic (C₅ to C₈) and/or aromatic (C₆ to C₁₂) radical which can optionally contain one or more oxygen bridges, sulphur bridges and/or -NR³ bridges,
R³ having the meaning given above,
and optionally an initiator, for the preparation of adhesive agents for use in a process for the therapeutic treatment of collagen-containing human and animal intercellular supporting substances.

12. Use according to Claim 11, after conditioning of the collagen-containing material with a fluid of a pH value from 0.1 to 3.5.

13. Use according to Claims 11 and 12 as an adhesive for fixing tooth repair materials to the tooth.

14. Use according to Claim 11 as an adhesive in bone cement.

## Claims (Claims for the following Contracting State(s): ES, GR)

1. Process for preparing adhesive formulations for use in a process for the therapeutic treatment of collagen-containing human and animal intercellular supporting substances, characterized in that the (meth) acrylic acid ester, containing formamide groups, of the formula 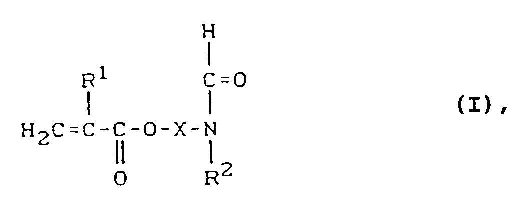 in which
R¹ denotes hydrogen or methyl,
R² denotes hydrogen, optionally hydroxy-, carboxy-, halogen- or amino-substituted (amino of the formula 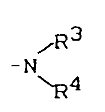 in which
R³ and R⁴ are identical or different and denote hydrogen or lower alkyl)
alkyl (C₁ to C₁₂), aryl (C₆ to C₁₂) or aralkyl (C₇ to C₁₄), and
X denotes a divalent, optionally hydroxy-, carboxy-, halogen- or amino-substituted (amino of the formula 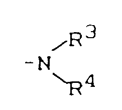 in which
R³ and R⁴ have the meaning given above)
aliphatic (C₁ to C₂₄) and/or cycloaliphatic (C₅ to C₈) and/or aromatic (C₆ to C₁₂) radical which can optionally contain one or more oxygen bridges, sulphur bridges and/or -NR³ bridges,
R³ having the meaning given above,
is mixed, with the further components of the formulation.

## Revendications (Revendications pour l'(les) Etat(s) contractant(s) suivant(s): AT, BE, CH, DE, FR, GB, IT, LI, LU, NL, SE)

1. Préparation d'adhésif contenant des esters (méth)acryliques contenant des groupes formamide répondant à la formule 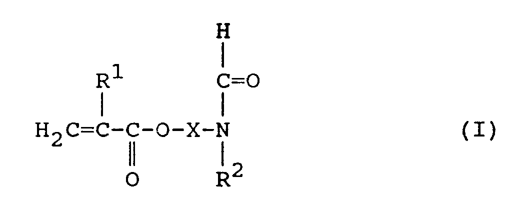 dans laquelle
R¹ représente un atome d'hydrogène ou un groupe méthyle,
R² représente un atome d'hydrogène, un groupe alkyle en C₁-C₁₂, un groupe aryle en C₆-C₁₂ ou un groupe aralkyle en C₇-C₁₄ éventuellement substitué par un groupe hydroxyle, par un groupe carboxyle, par un atome d'halogène ou par un groupe amino de formule 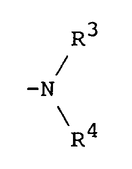 dans laquelle
R³ et R⁴ sont identiques ou différents et désignent un atome d'hydrogène ou un groupe alkyle inférieur, et
X représente un radical bivalent aliphatique en C₁-C₂₄ et/ou cycloaliphatique en C₅-C₈ et/ou aromatique en C₆-C₁₂ éventuellement substitué par un groupe hydroxyle, par un groupe carboxyle, par un atome d'halogène ou par un groupe amino de formule 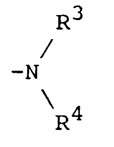 dans laquelle
R³ et R⁴ ont la signification mentionnée ci-dessus,
qui peut éventuellement contenir un ou plusieurs ponts oxygène, soufre et/ou NR³,
où
R³ a la signification mentionnée ci-dessus,
et éventuellement des initiateurs,
pour être utilisée dans un procédé pour le traitement thérapeutique de substances intercellulaires de support humaines et animales contenant du collagène.

2. Préparation selon la revendication 1, contenant des esters (méth)acryliques contenant des groupes formamide,
dans laquelle
R¹ représente un atome d'hydrogène ou un groupe méthyle,
R² représente un atome d'hydrogène, un groupe alkyle en C₁-C₆ éventuellement substitué par un groupe hydroxyle, par un groupe carboxyle, par un atome de fluor, par un atome de chlore ou par un groupe amino, un groupe phényle, un groupe tolyle, un groupe benzyle ou un groupe benzyle substitué par un groupe méthyle, et
X représente un radical bivalent aliphatique en C₁-C₂₀ et/ou cycloaliphatique en C₅-C₆ et/ou aromatique en C₆-C₁₂ éventuellement substitué par un groupe hydroxyle, par un groupe carboxyle, par un atome de fluor, par un atome de chlore ou par un groupe amino, qui peut éventuellement contenir de 1 à 10 ponts oxygène ou NR³,
où R³ a la signification mentionnée ci-dessus.

3. Préparation selon les revendications 1 et 2, contenant des esters (méth)acryliques contenant des groupes formamide,
dans laquelle
R¹ représente un atome d'hydrogène ou un groupe méthyle,
R² représente un atome d'hydrogène, un groupe alkyle en C₁-C₆ éventuellement substitué par un groupe hydroxyle, par un groupe carboxyle, par un atome de fluor, par un atome de chlore ou par un groupe amino, un groupe phényle ou un groupe tolyle, et
X représente un radical bivalent aliphatique en C₁-C₈ et/ou cycloaliphatique en C₅-C₆ et/ou phénylène éventuellement substitué par un groupe hydroxyle, par un groupe carboxyle, par un atome de fluor, par un atome de chlore ou par un groupe amino, qui peut éventuellement contenir de 1 à 4 ponts oxygène.

4. Préparation selon les revendications 1 à 3, caractérisée en ce que, comme initiateur, on met en oeuvre un formateur de radicaux de la série des composés mono- ou dicarbonyle.

5. Préparation selon les revendications 1 à 4, caractérisée en ce que l'ester (méth)acrylique contenant des groupes formamide et l'initiateur sont dissous dans un solvant.

6. Préparation selon les revendications 1 à 5, caractérisée en ce qu'on ajoute un coactivateur.

7. Préparation selon les revendications 1 à 6, caractérisée en ce que, comme composant supplémentaire, on ajoute un composé carbonyle.

8. Préparation selon les revendications 1 à 7, caractérisée en ce que, comme composant supplémentaire, on ajoute des esters (méth)acryliques qui peuvent former des réticulations.

9. Préparation selon les revendications 1 à 8, caractérisée en ce que, comme composant supplémentaire, on ajoute une matière de charge.

10. Procédé pour la mise au point de préparations d'adhésifs pour les utiliser dans un procédé pour le traitement thérapeutique de substances intercellulaires de support humaines et animales contenant du collagène, caractérisé en ce qu'on mélange l'ester (méth)acrylique contenant des groupes formamide, de formule 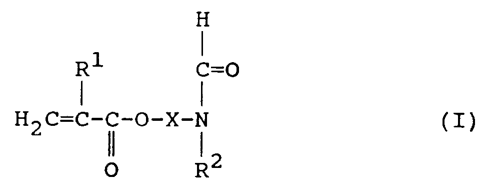 dans laquelle
R¹ représente un atome d'hydrogène ou un groupe méthyle,
R² représente un atome d'hydrogène, un groupe alkyle en C₁-C₁₂, un groupe aryle en C₆-C₁₂ ou un groupe aralkyle en C₇-C₁₄ éventuellement substitué par un groupe hydroxyle, par un groupe carboxyle, par un atome d'halogène ou par un groupe amino de formule 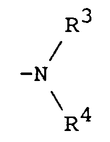 dans laquelle
R³ et R⁴ sont identiques ou différents et désignent un atome d'hydrogène ou un groupe alkyle inférieur, et
X représente un radical bivalent aliphatique en C₁-C₂₄ et/ou cycloaliphatique en C₅-C₈ et/ou aromatique en C₆-C₁₂ éventuellement substitué par un groupe hydroxyle, par un groupe carboxyle, par un atome d'halogène ou par un groupe amino de formule 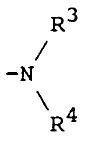 dans laquelle
R³ et R⁴ ont la signification mentionnée ci-dessus,
qui peut éventuellement contenir un ou plusieurs ponts oxygène, soufre et/ou NR³,
où
R³ a la signification mentionnée ci-dessus,
avec les autres composants de la préparation.

11. Utilisation de préparations contenant des esters (méth)acryliques contenant des groupes formamide, de formule 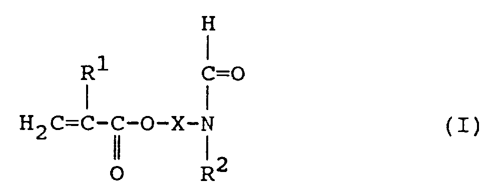 dans laquelle
R¹ représente un atome d'hydrogène ou un groupe méthyle,
R² représente un atome d'hydrogène, un groupe alkyle en C₁-C₁₂, un groupe aryle en C₆-C₁₂ ou un groupe aralkyle en C₇-C₁₄ éventuellement substitué par un groupe hydroxyle, par un groupe carboxyle, par un atome d'halogène ou par un groupe amino de formule 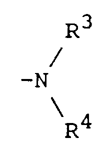 dans laquelle
R³ et R⁴ sont identiques ou différents et désignent un atome d'hydrogène ou un groupe alkyle inférieur, et
X représente un radical bivalent aliphatique en C₁-C₂₄ et/ou cycloaliphatique en C₅-C₈ et/ou aromatique en C₆-C₁₂ éventuellement substitué par un groupe hydroxyle, par un groupe carboxyle, par un atome d'halogène ou par un groupe amino de formule 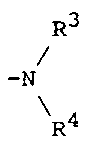 dans laquelle
R³ et R⁴ ont la signification mentionnée ci-dessus,
qui peut éventuellement contenir un ou plusieurs ponts oxygène, soufre et/ou NR³,
où
R³ a la signification mentionnée ci-dessus,
et éventuellement un initiateur, pour la préparation d'agents adhésifs pour les utiliser dans un procédé pour le traitement thérapeutique de substances intercellulaires de support humaines et animales contenant du collagène.

12. Utilisation selon la revendication 11, après un conditionnement de la matière contenant du collagène, à l'aide d'un liquide ayant une valeur de pH de 0,1 à 3,5.

13. Utilisation selon les revendications 11 et 12, comme adhésif pour la fixation de matières de réparation dentaire sur la dent.

14. Utilisation selon la revendication 11, comme agent adhésif dans du ciment d'os.

## Revendications (Revendications pour l'(les) Etat(s) contractant(s) suivant(s): ES, GR)

1. Procédé pour la mise au point de préparations d'adhésifs pour les utiliser dans un procédé pour le traitement thérapeutique de substances intercellulaires de support humaines et animales contenant du collagène, caractérisé en ce qu'on mélange l'ester (méth)acrylique contenant des groupes formamide, de formule 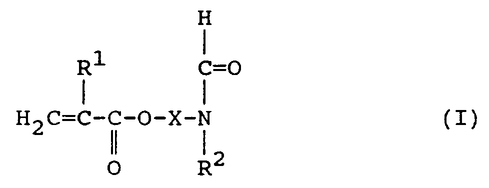 dans laquelle
R¹ représente un atome d'hydrogène ou un groupe méthyle,
R² représente un atome d'hydrogène, un groupe alkyle en C₁-C₁₂, un groupe aryle en C₆-C₁₂ ou un groupe aralkyle en C₇-C₁₄ éventuellement substitué par un groupe hydroxyle, par un groupe carboxyle, par un atome d'halogène ou par un groupe amino de formule 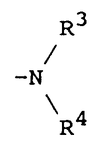 dans laquelle
R³ et R⁴ sont identiques ou différents et désignent un atome d'hydrogène ou un groupe alkyle inférieur, et
X représente un radical bivalent aliphatique en C₁-C₂₄ et/ou cycloaliphatique en C₅-C₈ et/ou aromatique en C₆-C₁₂ éventuellement substitué par un groupe hydroxyle, par un groupe carboxyle, par un atome d'halogène ou par un groupe amino de formule 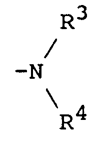 dans laquelle
R³ et R⁴ ont la signification mentionnée ci-dessus,
qui peut éventuellement contenir un ou plusieurs ponts oxygène, soufre et/ou NR³,
où
R³ a la signification mentionnée ci-dessus,
avec les autres composants de la préparation.
